# EUROPÄISCHE PATENTSCHRIFT

(11) **EP 0 879 620 B1**
(45) Veröffentlichungstag und Bekanntmachung des Hinweises auf die Patenterteilung: **10.12.2003**
(21) Anmeldenummer: 98250154.6
(22) Anmeldetag: 06.05.1998
(51) Int. Cl.: A61N 1/368

(54) **Vorrichtung zur Detektion einer Tachykardie**
Device for detection of a tachycardia
Dispositif pour la détection d'une tachycardie

(30) Priorität: 07.05.1997 US 852872
(43) Veröffentlichungstag der Anmeldung: 25.11.1998
(73) Patentinhaber: BIOTRONIK Mess- und Therapiegeräte GmbH & Co Ingenieurbüro Berlin, 12359 Berlin (DE)
(72) Erfinder: Nigam, Indra B., Lake Oswego, Oregon 97035 (US)
(74) Vertreter: Eisenführ, Speiser & Partner

(56) Entgegenhaltungen:
- EP-A- 0 469 817
- US-A- 5 545 186

## Beschreibung

Die Erfindung betrifft eine Vorrichtung der im Oberbegriff des Anspruchs 1 genannten Art.

Eine solche Vorrichtung ist als eigenständiges Diagnosegerät oder als Bestandteil eines implantierbaren Herzschrittmachers mit Antitachykardie-Betriebsart, eines Defibrillators, Kardioverters oder eines Kombinationsgerätes mit der Funktion eines der vorgenannten Geräte ausführbar.

Die Therapie tachykarder Herzrhythmusstörungen ist eines der wichtigen Aufgabengebiete der Kardiologie und speziell ein Hauptanwendungsfeld der Elektrostimulation des Herzens.

Angesichts der Vielfalt der Erscheinungsformen und Ursachen derartiger Störungen sind in den letzten Jahren umfangreiche Anstrengungen unternommen worden, die Voraussetzungen für einen therapeutischen Erfolg durch eine sichere Erkennung und Klassifizierung der tachykarden Arrhythmien zu verbessern.

Insbesondere besteht dabei das Problem der Entscheidung über das tatsächliche Vorliegen einer pathologischen Tachykardie im Gegensatz zu einer physiologischen Ratenerhöhung. Dies ist von besonderer Bedeutung, da im Falle einer fehlerhaften Diagnose durch eine falsche Therapie derartige pathologische Tachykardien durch Stimulation auch erst hervorgerufen werden können.

Es sind verschiedenste Vorrichtungen bzw. Verfahren zur Tachykardieerkennung bekannt.

So ist es aus der EP-A-0 094 758 bekannt, aus dem Vergleich eines aktuellen Zeitintervalls zwischen aufeinanderfolgenden Herzschlägen mit einem vorher bestimmten Mittelwert einerseits und mit einem vorgegebenen (Fest-)Wert andererseits ein Kriterium für das Vorliegen einer pathologischen Tachykardie zu gewinnen. Damit wurde gegenüber früheren Lösungen, die sich ausschließlich auf die Auswertung der aktuellen Herzrate stützten und dabei physiologische nicht von pathologischen Tachyarrhythmien unterscheiden konnten, ein Fortschritt erzielt. Für eine exakte Klassifizierung der verschiedenen Tachykardietypen reicht die Aussagekraft dieses Verfahrens jedoch nicht aus.

Später ist verschiedentlich - wie etwa aus der EP-A-0 302 577 hervorgeht - die Signalform des (auf konventionelle Weise oder intrakardial) aufgenommenen EKG als Unterscheidungs- und sogar Vorhersagekriterium vorgeschlagen worden. Die EKG-Signalform widerspiegelt jedoch das Auftreten einer ventrikulären Tachykardie nicht hinreichend verläßlich, und die zu einer einigermaßen aussagefähigen Auswertung benötigte hohe Signalauflösung erfordert einen sehr hohen Meß- und Verarbeitungsaufwand.

Eine ganze Reihe anderer Lösungsansätze bedient sich der Analyse der räumlichen Ausbreitung bzw. Korrelation von Depolarisationen im Herzgewebe. Dies erfordert die Implantation einer Vielzahl von Elektroden zur Signalerfassung - zum Teil sogar perikardial - und hat schon aus diesem Grunde nur geringe Realisierungschancen.

Aus der US-A-4 860 749 ist ein Verfahren zur Unterscheidung einer ventrikulären von einer Sinus- bzw. anderen supraventrikulären Tachykardie bekannt, bei dem die atriale und die ventrikuläre Herzrate (PP- bzw. RR-Intervall) sowie das AV-Intervall gemessen werden. Liegt das RR-Intervall innerhalb eines vorbestimmten Bereiches und ist es kürzer als das PP-Intervall, wird der Zustand ohne weiteres als ventrikuläre Tachykardie klassifiziert. Sind die atriale und die ventrikuläre Rate infolge 1:1-AV-Überleitung oder retrograder Überleitung etwa gleich, wird das gemessene AV-Intervall einem Vergleich mit einem vorgegebenen Wert ("Sinus-AV-Intervall") unterzogen und aus dem Ergebnis des Vergleichs das Klassifizierungskriterium gewonnen.

In der US-Patentschrift Nr. 5 327 900 ist ein Verfahren zur Unterscheidung zwischen pathologischen und physiologischen Tachykardien bei vergleichbarer atrialer und ventrikulärer Rate beschrieben, das auf der Zuordnung des gemessenen AV-Intervalls zu einem vorgegebenen AV-Zeitfenster beruht, welches anhand des AV-Intervalls bei normalem Sinusrhythmus bestimmt worden ist.

Ein weiteres Verfahren, bei dem zur Ermittlung der Therapierbarkeit einer festgestellten Tachyarrhythmie im Falle annähernder Übereinstimmung zwischen atrialer und ventrikulärer Rate eine ganze Reihe von zusätzlichen Kriterien (zum RR-Intervall und zum Verhältnis zwischen PP- und RR-Intervall) angewandt wird, ist in der US-A-5 379 776 beschrieben.

Dokument US-A-5 545 186 offenbart eine Vorrichtung gemäß des Oberbegriffs von Anspruch 1.

Der Erfindung liegt die Aufgabe zugrunde, eine relativ einfach aufgebaute und zu implantierende und stromsparend arbeitende Vorrichtung anzugeben, die eine pathologische Tachykardie mit großer Sicherheit und Schnelligkeit anzeigt.

Die Aufgabe wird durch eine Vorrichtung mit den Merkmalen des Anspruchs 1 gelöst.

Die Erfindung schließt den Gedanken ein, eine Vorrichtung zur Detektion einer pathologischen Tachykardie, insbesondere zum Einsatz mit einer implantierbaren Vorrichtung zur Terminierung der Tachykardie, zu schaffen, bei der die Erkennung auf der Grundlage von mehreren innerhalb eines Herzintervalls gleichzeitig zu prüfenden Kriterien erfolgt. Mit "gleichzeitiger" Prüfung ist dabei gemeint, daß diese Kriterien im wesentlichen innerhalb eines einzigen Herzzyklus gemeinsam angewendet werden. Dies kann dabei mit Mitteln der Prozessortechnik selbstverständlich auch zeitlich schnell nacheinander erfolgen. Von Bedeutung ist lediglich, daß hier die Ergebnisse unverzüglich gemeinsam zur Verfügung stehen und auf Schlag-zu-Schlag-Basis zusammenhängend korreliert werden können.

Hierfür sind insbesondere ein Zähler und Mittel zur differenzierten Veränderung des Zählerstandes in Abhängigkeit von den Ergebnissen der Prüfung der einzelnen Kriterien vorgesehen. Grundsätzlich erfolgt eine Heraufsetzung des Zählerstandes jeweils um einen vorgegebenen Zählbetrag, wenn innerhalb eines Herzintervalls mindestens eines der das Vorliegen eines Kriteriums anzeigenden primären Klassifizierungssignale aufgetreten ist. Weiter sind Ausgabemittel zur Abgabe eines das Auftreten einer Tachykardie kennzeichnenden Signals dann, wenn ein vorgegebener Zählerstand überschritten wird, vorgesehen.

In vorteilhafter Weiterbildung sind weiterhin Mittel zur Beibehaltung oder Herabsetzung des Zählerstands vorgesehen, wenn innerhalb des Herzintervalls keines der für das Auftreten einer Tachykardie maßgeblichen Kriterien erfüllt wurde. Insbesondere wird der Zähler nur dann dekrementiert, wenn mindestens zwei Herzintervalle aufeinanderfolgten, in denen keines der Kriterien für das Anstehen von Tachykardie erfüllt wurde.

Weiterhin ist vorteilhafterweise der Zählbetrag, um den der Zähler bei Erfüllung eines oder mehrerer Kriterien heraufgesetzt wird, jeweils größer als der Zählbetrag, um den der Zähler im Falle der Nichterfüllung herabgesetzt wird.

Bei einer anderen vorteilhaften Weiterbildung wird der Zählbetrag, um den der Zähler dekrementiert wird, so gewählt, daß er jeweils mit dem Abstand zweier Herzschläge abnimmt. Für die Ermittlung eines aktuellen Zählbetrags, um den der Zähler dekrementiert wird, ist insbesondere ein Hilfs-Zähler vorgesehen, welcher mit dem Beginn eines Herzintervalls gestartet und mit dessen Ende angehalten wird, wobei der jeweils erreichte Zählerstand ein Maß für den Zählbetrag bildet, um den der Zählerstand herabgesetzt wird.

Schließlich wird in einer bevorzugten Ausführung der Zähler jeweils auf Null gesetzt, wenn für eine vorgegebene Anzahl von aufeinanderfolgenden Herzschlägen keine erhöhte Herzrate festgestellt wurde.

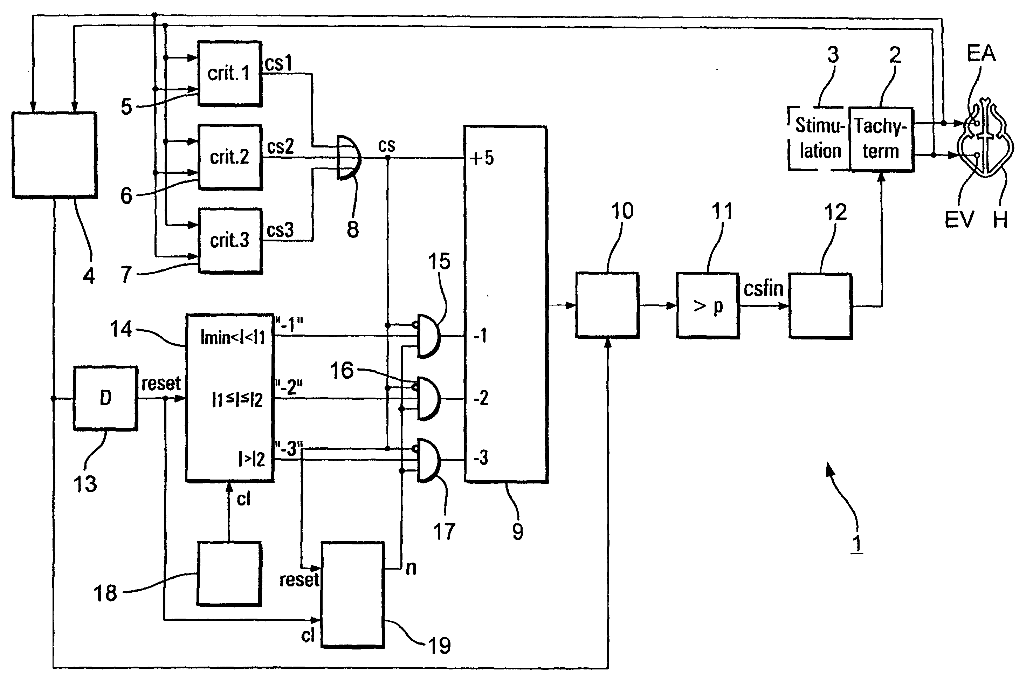

Vorteilhafte Weiterbildungen der Erfindung sind im übrigen in den Unteransprüchen gekennzeichnet bzw. werden nachstehend zusammen mit der Beschreibung der bevorzugten Ausführung der Erfindung anhand der einzigen Figur näher dargestellt.

Die Figur zeigt ein Funktions-Blockschaltbild einer Vorrichtung nach einer bevorzugten Ausführungsform der Erfindung.

In der Figur ist schematisch der Aufbau einer mit dem Herzen H über eine atriale und eine ventrikuläre Abfühlelektrode EA, EV verbundenen Vorrichtung 1 zur Tachykardieerkennung und -klassifizierung anhand ihrer im Zusammenhang mit der Erfindung wesentlichen Funktionselemente dargestellt.

Die Vorrichtung 1 ist verbunden mit einer Tachykardieterminierungseinheit 2, welche zusammen mit der Tachykardieerkennungsvorrichtung 1 implantierbar ausgestaltet ist. Sie umfaßt eine als Block 3 gestrichelt dargestellte Stimulationseinrichtung, welche beispielsweise den Generatorteil für eine kombinierte implantierbare Defibrillations- und Herzstimulationsvorrichtung bildet. Derartige Kombinationen sind grundsätzlich bekannt und sollen hier lediglich ein Beispiel für die vielseitige Anwendbarkeit der erfindungsgemäßen Vorrichtung bilden.

Die übrigen Baugruppen bilden funktionell eine Erfassungsund Auswertungseinheit für vom Herzen aufgenommene elektrische Signale, welche zur Tachykardieerkennung dienen und, wenn eine Tachykardie sicher erkannt ist, durch Ausgabe eines Steuersignals die Einleitung einer Stimulation zur Tachykardieterminierung über die Baugruppe 2 einleiten. Hierunter wird ggfs. auch eine Defibrillation verstanden.

Über die Elektroden EA, EV werden die Signale vom Herzen 1 über an sich bekannte (nicht dargestellte) Verstärkungsund Signalformungsmittel zu einem Herzschlagdetektor 4 weitergegeben, welcher Erfassungszyklen in Synchronisation zum Herzschlagverhalten (den Herzintervallen) steuert. Bei dem hier dargestellten einfachen Ausführungsbeispiel werden über den Herzschlagdetektor 4 P- bzw. R-Zacken erkannt, welche bei natürlichen oder stimulierten Herzschlägen über die Herzrate Aufschluß geben und somit auch physiologisch bedingte Anstiege der Herzfrequenz erkennen lassen.

Der Herzschlagdetektor steuert nicht nur die Erfassungszyklen, sondern dient zugleich zur Erkennung tachykarde Tendenzen anhand der Herzrate (der PP- oder RR-Intervalle).

Eien stark erhöhte Herzrate (Tachykardie) kann bekanntlich physiologischer Natur sein. Daher ist zusätzlich die Anwendung von zur Unterscheidung zwischen physiologischer und pathologischer Tachykardie dienenden Tachykardiekriterien vorgesehen.

Die vom Herzen H über die Elektroden E_{A}, E_{V} abgeleiteten Signale werden hierzu parallel drei Klassifizierungseinheiten 5 bis 7 zugeführt, welche jeweils das Vorliegen eines von drei vorbestimmten Kriterien für eine pathologische Tachykardie crit1 bis crit3, die weitgehend vom aktuellen Ratenwert unabhängig sind, prüfen und somit das Auftreten einer pathologischen Tachykardie detektieren. Jede Klassifizierungsbaugruppe 5 bis 7 gibt ein primäres Klassifizierungssignal cs1 bis cs3 aus. Die primären Klassifizierungssignale werden einem Oder-Gatter 8 zugeführt.

Diese anzuwendenden Tachykardiekriterien können zum Teil aus dem Stand der Technik, zum anderen Teil aber im einzelnen auch aus einer parallel eingereichten Patentanmeldung derselben Anmelderin entnommen werden.

Es handelt sich bei einem dieser Kriterien beispielsweise um die Schnelligkeit von Änderungen der ventrikulären Herzrate (des RR-Intervalls) im Vergleich zu einem vorangehenden Herzintervall. In einer entsprechenden Ausführung wird in der ersten Klassifizierungsbaugruppe 5 beispielsweise geprüft, ob die Tachyarrhythmie plötzlich eingesetzt hat. Hierzu wird der aktuell ermittelte RR-Intervall-Mittelwert (siehe dazu weiter unten) einem Vergleich mit einem vorhergehenden Mittelwert unterzogen. Das von der Baugruppe 5 abgegebene Ausgangssignal drückt dann aus, ob eine den gespeicherten zulässigen Differenzbetrag übersteigende Verkürzung des RR-Intervalls vorliegt, die als Beleg für ein plötzliches Einsetzen der Tachyarrhythmie zu werten ist. Ein solches ist ein Indiz für das Vorliegen einer ventrikulären (pathologischen) Tachykardie oder Fibrillation.

Ein weiteres Kriterium ist das Verhältnis der Länge der RR-Intervalle (Mittelwerte) zur Länge der PP-Intervalle (Mittelwerte). Sind die RR-Intervalle kürzer als die PP-Intervalle, so spricht dies für das Vorliegen einer ventrikulären Tachykardie. Entsprechend wird also z.B. die zweite Klassifizierungsbaugruppe 6 Mittel zur Bestimmung des PP-Intervalls - bevorzugt eines Mittelwertes hiervon über mehrere Erfassungszyklen - , Mittel zur Quotientenbildung aus dem RR- und dem PP-Intervallwert bzw. -mittelwert und eine nachgeordnete Diskrimatorstufe aufweisen.

Ein weiteres Klassifikationskriterium ist die zeitliche Stabilität der RR-Intervalle. Entsprechend werden z.B. in der Klassifizierungsbaugruppe 7 die RR-Intervallwerte einem Vergleich mit einem programmierten Stabilitätskriterium unterzogen. (In praxi werden bevorzugt die Differenzen zwischen dem aktuellen und jedem der drei vorhergehenden RR-Intervall-Mittelwerte mit dem Stabilitätskriterium verglichen, und Stabilität wird als gegeben angenommen, wenn in allen drei Vergleichen das Kriterium erfüllt ist.) Sind sie stabil, so wird zusätzlich geprüft, ob der RR-Intervall-Mittelwert ein ganzzahliges Vielfaches des (in der Baugruppe 6 verfügbaren) PP-Intervall-Mittelwertes ist. Wird festgestellt, daß der RR-Mittelwert ein ganzzahliges Vielfaches des PP-Mittelwertes ist, handelt sich dann um eine n:1 in den Ventrikel übergeleitete atrielle Tachykardie. Wird hingegen festgestellt, daß er kein ganzzahliges Vielfaches ist ("Nein" im Schritt S8), spricht dies für eine ventrikuläre Tachykardie.

Weitere anwendbare Kriterien sind die Regularität der PR-Intervalle und/oder die Stabilität der PP-Intervalle oder die Monotonie der zeitlichen Änderung der PR-Intervalle. Dies Kriterien können in zusätzlichen Klassifizierungsbaugruppen oder anstelle eines der oben spezifizierten Kriterien in den Baugruppen 5 bis 7 angewandt werden.

An dieser Stelle ist zu vermerken, daß die Darstellung der Klassifizierungsbaugruppen in hardwaremäßiger Ausgestaltung hier lediglich der Verdeutlichung des Prinzips dient. Es ist klar, daß hierbei auch Softwarealgorithmen verwendet werden können, welche die Anwendung der Tachykardiekriterien auch unmittelbar nacheinander innerhalb eines Meßzyklus realisieren.

Das Ausgangssignal cs des Oder-Gatters 8 wird einem Inkrementierungseingang "+5" eines Zählers 9 zugeführt, welcher bei Erfüllung eines der Tachykardiekriterien um einen vorbestimmten Wert heraufgesetzt wird. Dieser Wert ist hier mit 5 angegeben. Es können auch andere Inkrementierungswerte verwendet werden, wobei wichtig ist, daß durch die Wahl des Inkrementierungswertes eine Gewichtung der den Zähler 9 beeinflussenden Ereignisse herbeigeführt wird.

Über eine dem Zähler 9 nachgeschaltete Torschaltung 10 wird bei Erfassung eines folgenden Herzschlags (als Abschluß des Erfassungszyklus), ausgehend vom Herzschlagdetektor 4 über einen Aktivierungeingang der Torschaltung 10, der Zählerstand des Zählers 9 an eine Diskriminatorstufe 11 gegeben. Diese gibt ein Ausgangssignal csfin ab, wenn der Stand des Zählers 9 einen vorgegebenen Wert p überschreitet. Daraufhin wird durch die Steuersignal-Ausgangsstufe 12 ein Stimulationsmuster ausgelöst, welches durch die Tachykardieterminierungseinheit 2 abgegeben wird und die Tachykardie terminieren soll. Liegt der Zählerstand des Zählers 9 unterhalb von p, gibt die Diskriminatorstufe 11 kein Ausgangssignal ab, und es erfolgt keine Stimulation zur Tachykardieterminierung.

Wenn über die Klassifizierungsbaugruppen 5 bis 7 ein Zustand erkannt wird, der keines der Kriterien crit1 bis crit3 erfüllt, wird der Zähler 9 unter festgelegten Voraussetzungen und auf vorbestimmte Weise dekrementiert.

Zur Bestimmung des anzuwendenden Dekrementierungsbetrages wird in den (unten genauer beschriebenen) Baugruppen 13, 14 und 18 die aktuelle Herzrate bestimmt und klassifiziert. In Abhängigkeit davon, ob die Herzrate in einen bradykarden Bereich, Normalbereich oder tachykarden Bereich fällt, wird der Zähler mit unterschiedlichen Beträgen dekrementiert. Beim vorliegenden Beispiel werden drei verschiedene Dekrementierungswerte "-1", "-3" und "-2" in Abhängigkeit davon angewandt, ob der aktuelle Ratenwert f oberhalb eines ersten Raten-Schwellwertes f1, unterhalb eines zweiten Raten-Schwellwertes f2 oder zwischen den Werten f1 und f2 liegt. (Natürlich sind auch andere Werte-Konstellationen anwendbar, einschließlich der Möglichkeit, den erreichten Zählerstand beizubehalten.)

Auf diese Weise wird erreicht, daß die Bereitschaft der Vorrichtung, in eine Tachykardieterminierung erneut einzutreten, erhöht bleibt, wenn die Herzrate erhöht ist, d.h. die Herzschläge in einen tachykarden Bereich fallen. Ist die Herzschlagrate bradykard, so wird diese Bereitschaft durch Dekrementieren des Zählers 9 mit den höchsten Dekrementbeträgen relativ schnell abgebaut und es muß tendenziell eine Anzahl von Erfassungszyklen durchlaufen sein, in denen eine der Stufen 5 bis 7 angesprochen hat, um wieder eine Tachykardieterminierung auszulösen. Auf diese Weise paßt sich das System in seiner Reaktionsbereitschaft dem Herzverhalten aufgrund vergangener Ereignisse an, um eine Tachykardieterminierung erst dann einzuleiten, wenn tatsächlich viele Anzeichen für eine entsprechendes Notwendigkeit sprechen.

Zur Realisierung dieser Funktion wird über einen Intervallzähler und -diskriminator 14, der von einem Taktgeber 18 angesteuert und jeweils nach einem über die Baugruppe 4 detektierten Herzschlag - verzögert über das D-Glied 13 - zurückgesetzt wird, die durch einen Zählwert ausgedrückte Länge l des letzten Herzschlagintervalls einem der vorbestimmten Bereiche "1 > 12" (entsprechend der Herzrate f > f1), "11 ≤ 1 ≤ 12" (entsprechend f2 ≤ f ≤ f1) oder "1 > 12" (entsprechend f < f2) zugeordnet. Am jeweils aktivierten der drei Ausgänge wird ein der getroffenen Zuordnung entsprechendes Dekrementierungssignal "-1", "-2" oder "-3" ausgegeben.

Wenn der Intervallzähler und -diskriminator durch ein Signal des Herzschlagdetektors 4 zurückgesetzt wird, bevor ein minimaler Zählerstand erreicht ist, so wird kein Ausgangssignal abgegeben. Der Zähler 9 wird daraufhin unter der Voraussetzung, daß keine der Schaltungen 5 bis 7 angesprochen hat, weder dekrementiert noch inkrementiert.

Die Ausgänge des Intervallzählers und -diskriminators 14 sind jeweils über ein Und-Gatter 15, 16 bzw. 17 mit einem Steuereingang des Zählers 9 verbunden. Eine Entsperrung des Und-Gatter 15 bis 17, d.h. eine Durchschaltung des durch den Intervallzähler und -diskriminator 14 gelieferten Dekrementierungssignals zum entsprechenden Eingang des Zählers 9 erfolgt unter folgenden Voraussetzungen:
(a) Am Ausgang des Oder-Gatters 8 liegt aktuell kein die Erfüllung eines der Kriterien crit1 bis crit3 anzeigendes Signal an, so daß die invertierenden Eingänge der Und-Gatter nicht aktiviert sind.
(b) Keine der Klassifizierungsbaugruppen 5 bis 7 hat in n vorangegangenen Meßzyklen angesprochen.

Die Erfüllung der Voraussetzung (b) wird durch einen weiteren Zähler 19 dokumentiert, welcher über den Herzschlagdetektor 4 und das D-Glied 13 getaktet ist und jeweils durch ein Ausgangssignal des Oder-Gatters 8 zurückgesetzt wird. Die Und-Gatter 15 bis 17 werden entsperrt, wenn der Zähler 19 einen vorgegebenen Zählerstand n erreicht hat. Da der Zähler 19 jeweils am Ende eines Erfassungszyklus vom Ausgang des D-Gliedes 13 her angesteuert wird, müssen hierfür n Erfassungszyklen vergangen sein, ohne daß eine der Detektions-Baugruppen 5 bis 7 angesprochen hat.

Die Erfindung beschränkt sich in ihrer Ausführung nicht auf die vorstehend angegebenen bevorzugten Ausführungsbeispiele. Vielmehr ist eine Anzahl von Varianten denkbar, welche von der dargestellten Lösung auch in anders gearteter Ausführung Gebrauch machen.

Insbesondere kann die Lösung hard- oder softwaremäßig oder in kombinierter Weise realisiert werden.

Das oben skizzierte Vorgehen und die gezeigte Anordnung können vielfältig abgewandelt sein, beispielsweise durch Verzicht auf den Zähler 19 und die Ausführung einer Dekrementierung des Zählers 9 auf einer Schlag-zu-Schlag-Basis (ohne Auswertung der Erfüllung von Tachykardiekriterien in früheren Erfassungszyklen).

Von besonderem Interesse ist auch eine gegenüber der obigen Darstellung modifizierte Verknüpfung der Klassifizierungsbaugruppen, bei der mindestens einige Und-Verknüpfungen vorliegen, so daß nur bei gleichzeitiger Erfüllung der so verknüpften (vorläufigen) Kriterien ein gültiges Klassifizierungssignal für eine pathologische Tachykardie ausgegeben wird.

Die Vorrichtung kann eine selbständige Einheit sein oder im Verbund mit anderen (speziell implantierbaren) Geräten, insbesondere einem Herzschrittmacher oder Schrittmacher/Defibrillator-Kombinationsgerät, eingesetzt werden.

## Patentansprüche

1. Vorrichtung (1) zur Detektion einer pathologischen Tachykardie, insbesondere zum Einsatz mit einer implantierbaren Vorrichtung (2) zur Tachykardieterminierung, mit einer auf mindestens ein vom Herzen abgeleitetes elektrisches Signal ansprechenden und mindestens ein Klassifizierungskriterium verarbeitenden Klassifizierungseinrichtung, die bei Erfüllung des Klassifizierungskriteriums ein eine pathologische Tachykardie anzeigendes Ausgangssignal (csfin) abgibt, und mit
einer Mehrzahl von Klassifizierungseinrichtungen (5 bis 7) zur Prüfung von verschiedenen Klassifizierungskriterien (crit1 bis crit3) innerhalb eines Herzintervalls oder aufgrund der Auswertung von vorangehenden Herzintervallen, welche jeweils bei Erfüllung eines der Klassifizierungskriterien eines von mehreren primären Klassifizierungssignalen (cs1 bis cs3) abgeben,
**gekennzeichnet durch**
einen über erste logische Mittel (8) mit den Ausgängen der Klassifizierungseinrichtungen verbundenen steuerbaren Zähler (9),
Mittel (8) zum Inkrementieren des Zählerstandes des Zählers jeweils um einen vorgegebenen Zählbetrag, wenn während eines Herzintervalls mindestens eines der primären Klassifizierungssignale erscheint,
Mittel zur Konstanthaltung oder Dekrementierung des Zählerstands um einen an deren vorgegebenen Zählbetrag, wenn innerhalb des Herzintervalls kein primäres Klassifizierungssignal erscheint, und
Diskriminatormittel (11), welche das das Auftreten einer pathologischen Tachykardie anzeigende Ausgangssignal (csfin) abgeben, wenn der Zählerstand des Zählers (9) einen vorgegebenen Wert (p) überschreitet.

2. Vorrichtung nach Anspruch 1, **dadurch gekennzeichnet daß** der Zählbetrag, um den der Zähler (9) im Falle des Auftretens eines primären Klassifizierungssignals (c11 bis cl3) inkrementiert wird, größer ist als der oder jeder Zählbetrag, um den der Zähler dekrementiert wird, wenn innerhalb des Herzintervalls kein primäres Klassifizierungssignal erscheint.

3. Vorrichtung nach Anspruch 1 oder 2, **dadurch gekennzeichnet daß** der Zählbetrag, um den der Zähler (9) dekrementiert wird, mit zunehmendem Abstand zweier aufeinanderfolgender Herzschläge zunimmt.

4. Vorrichtung nach Anspruch 3, **dadurch gekennzeichnet daß** für die Ermittlung des Zählbetrags, um den der Zähler (9) dekrementiert wird, ein erster Hilfs-Zähler (14) vorgesehen ist, welcher jeweils mit dem Beginn eines Herzzyklus gestartet wird und dessen am Ende des Herzzyklus erreichter Zählerstand den Zählbetrag bestimmt, um den der Zähler dekrementiert wird.

5. Vorrichtung nach einem der vorangehenden Ansprüche, **dadurch gekennzeichnet daß** der Zähler (9) nur dann dekrementiert wird, wenn eine vorbestimmte Mehrzahl von Herzintervallen aufeinanderfolgen, in denen kein primäres Klassifizierungssignal aufgetreten ist.

6. Vorrichtung nach Anspruch 5, **gekennzeichnet durch** einen über die Klassifizierungseinrichtungen (6 bis 7) gesteuerten zweiten Hilfs-Zähler (19) zur Bestimmung der Anzahl der aufeinanderfolgenden Herzintervalle, in denen kein primäres Klassifizierungssignal aufgetreten ist.

7. Vorrichtung nach Anspruch 5 oder 6, **dadurch gekennzeichnet, daß** der Zähler (9) auf Null rückgesetzt wird, wenn mindestens zwei Herzintervalle aufeinanderfolgen, in denen kein primäres Klassifizierungssignal aufgetreten ist.

8. Vorrichtung nach einem der vorhergehenden Ansprüche, **dadurch gekennzeichnet, daß** die ersten logischen Mittel ein Oder-Gatter (8) aufweisen, mit dessen Eingängen die Ausgänge aller Klassifizierungseinrichtungen (5 bis 7) mindestens mittelbar verbunden sind.

9. Vorrichtung nach einem der vorhergehenden Ansprüche, **dadurch gekennzeichnet, daß** die ersten logischen Mittel ein Und-Gatter aufweisen, mit dessen Eingängen die Ausgänge mindestens eines Teils der Klassifizierungseinrichtungen verbunden sind.

10. Vorrichtung nach einem der Ansprüche 4 bis 9, **dadurch gekennzeichnet, daß** zwischen den Ausgang des ersten Hilfs-Zählers (14) und den zugeordneten Steuereingang des Zählers (9) zweite logische Mittel geschaltet sind, die ihrerseits über einen Steuereingang mit dem Ausgang der ersten logischen Mittel (8) verbunden sind.

## Claims

1. Apparatus (1) for the detection of a pathological tachycardia, in particular for use with an implantable apparatus (2) for tachycardia termination, comprising a classification device which is responsive to at least one electrical signal derived from the heart and which processes at least one classification criterion and which when the classification criterion is fulfilled delivers an output signal (csfin) indicating a pathological tachycardia, and a plurality of classification devices (5 to 7) for testing various classification criteria (crit1 to crit3) within a cardiac interval or on the basis of evaluation of preceding cardiac intervals which when one of the classification criteria is fulfilled respectively deliver one of a plurality of primary classification signals (cs1 to cs3),
**characterised by**
a controllable counter (9) connected by way of first logic means (8) to the outputs of the classification devices,
means (8) for incrementing the counter condition of the counter by a respective predetermined counting amount if at least one of the primary classification signals appears during a cardiac interval,
means for keeping constant or decrementing the counter condition by another predetermined counting amount if no primary classification signal appears within the cardiac interval, and
discriminator means (11) which deliver the output signal (csfin) indicating the occurrence of a pathological tachycardia if the counter condition of the counter (9) exceeds a predetermined value (p).

2. Apparatus according to claim 1 **characterised in that** the counting amount by which the counter (9) is incremented in the case of a primary classification signal (cl1 to cl3) occurring is greater than the or each counting amount by which the counter is decremented if no primary classification signal appears within the cardiac interval.

3. Apparatus according to claim 1 or claim 2 **characterised in that** the counting amount by which the counter (9) is decremented increases with increasing distance between two successive heartbeats.

4. Apparatus according to claim 3 **characterised in that** provided for ascertaining the counting amount by which the counter (9) is decremented is a first auxiliary counter (14) which is started in each case with the beginning of a cardiac cycle and whose counter condition, which is reached at the end of the cardiac cycle, determines the counting amount by which the counter is decremented.

5. Apparatus according to one of the preceding claims **characterised in that** the counter (9) is decremented only when a predetermined multiplicity of cardiac intervals in which no primary classification signal has appeared occur in succession.

6. Apparatus according to claim 5 **characterised by** a second auxiliary counter (19) controlled by way of the classification devices (6 to 7) for determining the number of successive cardiac intervals in which no primary classification signal has occurred.

7. Apparatus according to claim 5 or claim 6 **characterised in that** the counter (9) is reset to zero if at least two cardiac intervals in which no primary classification signal has appeared occur in succession.

8. Apparatus according to one of the preceding claims **characterised in that** the first logic means have an OR-gate (8), to the inputs of which the outputs of all classification devices (5 to 7) are at least indirectly connected.

9. Apparatus according to one of the preceding claims **characterised in that** the first logic means have an AND-gate, to the inputs of which the outputs of at least a part of the classification devices are connected.

10. Apparatus according to one of claims 4 to 9 **characterised in that** connected between the output of the first auxiliary counter (14) and the associated control input of the counter (9) are second logic means which in turn are connected by way of a control input to the output of the first logic means (8).

## Revendications

1. Dispositif (1) pour détecter une tachycardie pathologique, notamment destiné à être utilisé avec un dispositif implantable (2) pour déterminer une tachycardie, comportant au moins un dispositif de classification qui répond qui répond à un signal électrique délivré par le coeur et traite au moins un critère de classification et qui, lorsque le critère de classification est satisfait, délivre un signal de sortie (csfin) indiquant une tachycardie pathologique, et comportant
une multiplicité de dispositifs de classification (5 à 7) pour contrôler différents critères de classification (crit1 à crit3) pendant l'intervalle cardiaque ou sur la base de l'évaluation d'intervalles cardiaques précédents, qui délivrent un ou plusieurs signaux primaires de classification (cs1 à cs3) respectivement lorsque l'un des critères de classification est satisfait,
**caractérisé par**
un compteur commandable (9) relié aux sorties des dispositifs de classification par l'intermédiaire deux premiers moyens logiques (8),
des moyens (8) pour incrémenter l'état du compteur respectivement d'une valeur prédéterminée de comptage, lorsque, pendant un intervalle cardiaque, au moins l'un des signaux primaires de classification apparaît,
des moyens pour maintenir constant ou décrémenter l'état du compteur d'une autre valeur de comptage prédéterminé lorsque, pendant l'intervalle cardiaque, aucun signal primaire de classification n'apparaît, et
des moyens formant discriminateur (11), qui délivrent le signal de sortie (csfin) indiquant l'apparition d'une tachycardie pathologique, lorsque l'état de contact du compteur (9) dépasse une valeur prédéterminée (p).

2. Dispositif selon la revendication 1, **caractérisé en ce que** la valeur de comptage, dont le compteur (9) est incrémenté dans le cas de l'apparition d'un signal primaire de classification (c11 à c13), est supérieure à la ou à chaque valeur de comptage, dont le compteur est décrémenté lorsque dans l'intervalle cardiaque il n'apparaît aucun signal primaire de classification.

3. Dispositif selon la revendication 1 ou 2, **caractérisé en ce que** la valeur de comptage, dont le compteur (9) est décrémenté, augmente lorsque l'intervalle entre deux battements cardiaques successifs augmente.

4. Dispositif selon la revendication 3, **caractérisé en ce que** pour la détermination de la valeur de comptage, dont le compteur (9) est décrémenté, il est prévu un premier compteur auxiliaire (14), qui démarre respectivement au début d'un cycle cardiaque et dont l'état de comptage, atteint à la fin du cycle cardiaque, détermine la valeur de comptage, dont le compteur est décrémenté.

5. Dispositif selon l'une des revendications précédentes, **caractérisé en ce que** le compteur (9) est décrémenté uniquement lorsqu'une multiplicité prédéterminée d'intervalles cardiaques, dans lesquels aucun signal primaire de classification n'est apparu, se succèdent.

6. Dispositif selon la revendication 5, **caractérisé en ce qu'**un second compteur auxiliaire (19), qui est commandé au moyen des dispositifs de classification (6 à 7) et qui sert à déterminer le nombre des intervalles cardiaques successifs, dans lesquels aucun signal primaire de classification n'est apparu.

7. Dispositif selon la revendication 5 ou 6, **caractérisé en ce que** le compteur (9) est ramené à zéro lorsqu'au moins deux intervalles cardiaques, dans lesquels aucun signal primaire de classification n'est apparu, se succèdent.

8. Dispositif selon l'une des revendications précédentes, **caractérisé en ce que** les premiers moyens logiques comportent une porte OU (8) aux entrées de laquelle sont raccordées au moins de façon indirecte les sorties de tous les dispositifs de classification (5 à 7).

9. Dispositif selon l'une des revendications précédentes, **caractérisé en ce que** les premiers moyens logiques comportent une porte ET aux entrées de laquelle sont raccordées les sorties d'au moins une partie des dispositifs de classification.

10. Dispositif selon l'une des revendications 4 à 9, **caractérisé en ce qu'**entre la sortie du premier compteur auxiliaire (14) et l'entrée de commande associée du compteur (9) sont branchés des seconds moyens logiques, qui pour leur part sont raccordés, par l'intermédiaire d'une entrée de commande à la sortie des premiers moyens logiques (8) .
